# EUROPÄISCHE PATENTSCHRIFT

(11) **EP 0 925 766 B1**
(45) Veröffentlichungstag und Bekanntmachung des Hinweises auf die Patenterteilung: **14.05.2003**
(21) Anmeldenummer: 98890312.6
(22) Anmeldetag: 29.10.1998
(51) Int. Cl.: A61F 2/38, A61F 2/64, A61F 5/01

(54) **Gelenk (Kniegelenk)**
Body joint, especially knee joint
Articulation, en particulier pour le genou

(30) Priorität: 19.12.1997 AT 215097
(43) Veröffentlichungstag der Anmeldung: 30.06.1999
(73) Patentinhaber: GRAFINGER, Josef, A-1160 Wien (AT)
(72) Erfinder: GRAFINGER, Josef, A-1160 Wien (AT)
(74) Vertreter: Rippel, Andreas, Dipl.-Ing.

(56) Entgegenhaltungen:
- DE-C- 897 314
- FR-A- 1 136 136
- US-A- 4 655 201
- US-A- 5 358 527

## Beschreibung

Die Erfindung bezieht sich auf ein Gelenk entsprechend dem Oberbegriff des Anspruches 1.

Die Schwierigkeit bei der Konstruktion von verschiedenen Gelenken, insbesondere Kniegelenken, besteht vor allem darin, daß die natürliche Bewegung keine reine Drehbewegung, sondern eine kombinierte Dreh- und Gleitbewegung ist.

Aus der AT PS 393.620 ist ein Gelenk für eine Knieführungsschiene bekannt geworden, bei der in einer ersten Schiene zwei bogenförmige Führungen vorgesehen sind, in die den Schwenkwinkel der beiden Schienen begrenzende Zapfen der zweiten Schiene eingreifen. Die Zapfen oder Bolzen stellen dabei in der jeweiligen Endstellung die Achse für die kreisbogenförmige Führung des anderen Zapfens dar. Die natürliche Bewegung des Knies kann durch eine derartige Anordnung nicht nachgeahmt werden.

Aus der US-A-5 009 223 sind Gelenke für Knieführungsschienen bekannt geworden, bei denen zusätzlich zu den beiden bogenförmigen Führungen zwei weitere bogenförmige Führungen angeordnet sind. Diese beiden zusätzlichen Führungen bilden eine Art "T", wobei in einem Steg des "T" ein Bolzen, im anderen Steg des "T" ein weiterer Bolzen der anderen Schiene geführt ist Auch bei dieser bekannten Anordnung ist eine dem natürlichen Bewegungsablauf entsprechende Führung nicht möglich.

In der US-A-4 655 201 ist ein Gelenk mit den Merkmalen des Oberbegriffes des Anspruches 1 beschrieben. Durch dieses bekannte Kniegelenk ist zwar eine Annäherung an den natürlichen Bewegungsablauf möglich, aber eben nur eine Annäherung.

Die Erfindung hat es sich zum Ziel gesetzt, ein Gelenk der eingangs genannten Art zu schaffen, das eine kombinierte Dreh- und Gleitbewegung ermöglicht und insbesondere, aber nicht ausschließlich, als Ersatz von Kniegelenken eingesetzt werden kann. Es soll dabei auch die Möglichkeit gegeben sein, Verschleißteile verhältnismäßig einfach auszutauschen, wobei bei einem Austausch auch veränderte Bewegungsabläufe ermöglicht werden können.

Erreicht wird dies durch die kennzeichnenden Merkmale des Anspruches 1.

Eine derartige Anordnung ermöglicht eine kombinierte Dreh- und Gleitbewegung, die durch Veränderung der bogenförmigen Führungen den jeweiligen Gegebenheiten angepaßt werden kann. Auch ist ein Austausch der Verschleißteile verhältnismäßig einfach möglich.

Bei einer bevorzugten Ausführungsform der Erfindung besitzt der Nockenkörper im Querschnitt die Form eines Bogendreieckes mit abgerundeten Ecken.

Die vorspringenden zweiten Nocken können dabei an der der Schnittkante der beiden Zylinderflächen gegenüberliegenden Seite des Bogendreieckes ausgebildet sein.

Der Anschluß eines Schwenkteiles ist vorteilhaft gegenüberliegend der Schnittkante der beiden Zylinderflächen angeordnet

Zur Halterung der beiden Halbschalen werden diese von einem Halteteil teilweise umfaßt, an dem ein Anschluß für den anderen Schwenkteil angeordnet ist.

Die Halbschalen sind dabei in den Halteteil einschiebbar ausgebildet, sodaß sie leicht auswechselbar sind.

Nachstehend ist die Erfindung anhand eines in den Zeichnungen dargestellten Ausführungsbeispieles näher beschrieben, ohne auf dieses Beispiel beschränkt zu sein.

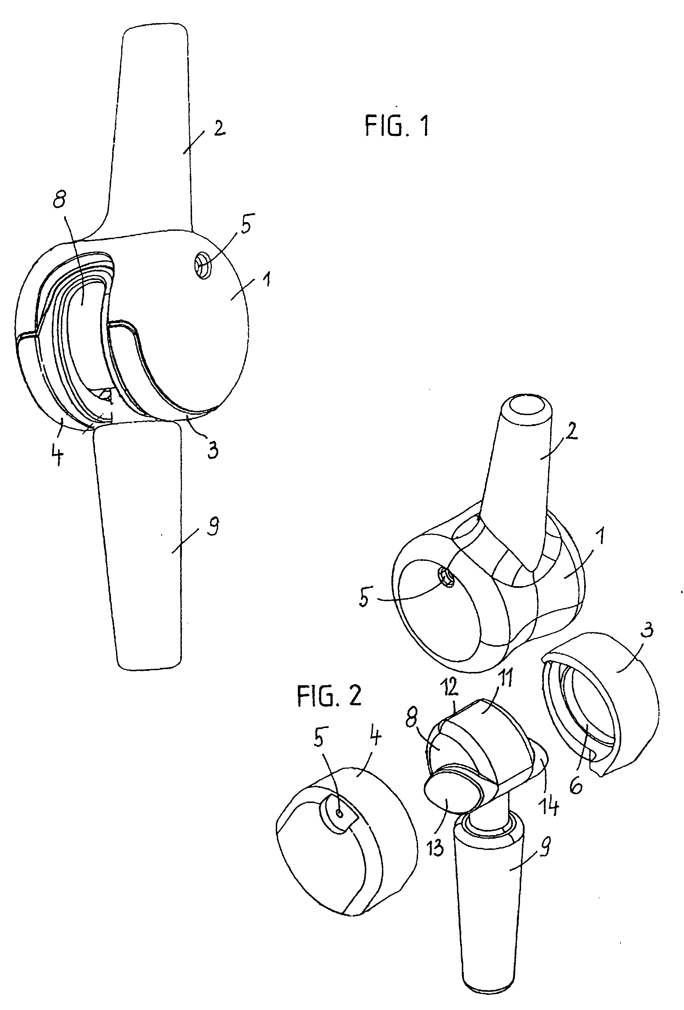

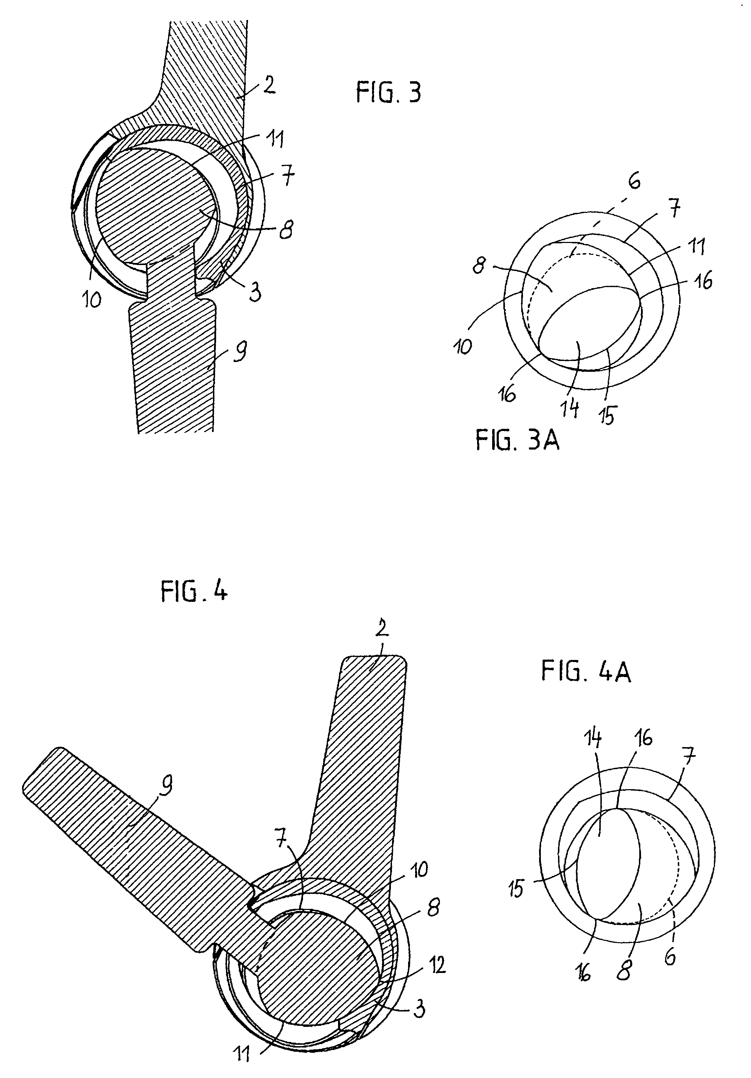

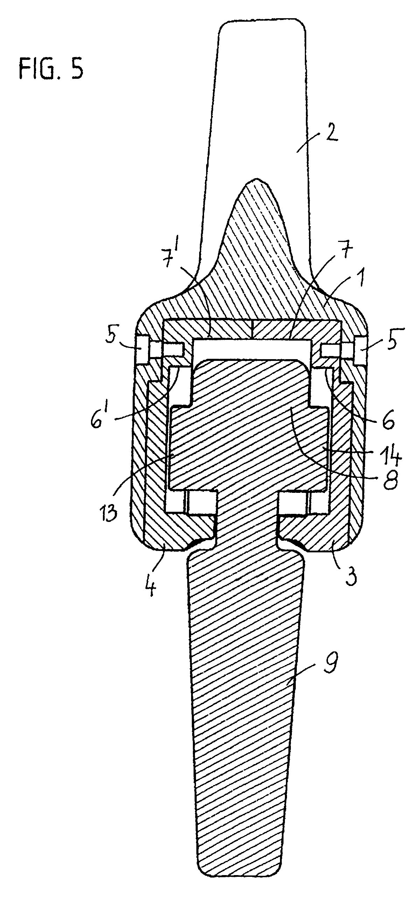

Dabei zeigen: Fig. 1 die schaubildliche Ansicht eines erfindungsgemäßen Gelenkes; Fig. 2 eine Explosionszeichnung des Gelenkes nach Fig. 1; Fig. 3 einen Längsschnitt durch das Gelenk in gestreckter Stellung; Fig. 3a die wesentlichen Führungsteile des Gelenkes in der Stellung nach Fig. 3; Fig. 4 einen Längsschnitt durch das Gelenk in der maximal verschwenkten Stellung; Fig. 4a die wesentlichen Führungsteile des Gelenkes in der Stellung nach Fig. 4; Fig. 5 einen Schnitt durch das Gelenk entlang einer zur Schnittebene der Fig. 3 senkrechten Schnittebene.

Das Ausführungsbeispiel nach den Zeichnungen stellt eine Knieprothese dar Bei dieser ist an einem Halteteil 1 ein Anschlußzapfen 2 angeordnet, der mit dem Oberschenkel verbindbar ist. In den Halteteil 1 sind zwei Halbschalen 3 und 4 eingeschoben, die über Bohrungen 5 und nicht dargestellte Schrauben am Halteteil 1 zu befestigen sind.

In den Halbschalen 3 und 4 sind bogenförmige Führungen 6, 6' und 7, 7' ausgebildet, wobei die Führungen 7, 7' gegenüber den Führungen 6, 6' abgestuft sind.

Die Halbschalen 3, 4 nehmen einen Nockenkörper 8 auf, an dem ein Zapfen 9 angeordnet ist, der mit dem Unterschenkel zu verbinden ist. Der Nockenkörper 8 besitzt im Querschnitt etwa die Form eines Bogendreieckes. Zwei Zylinderflächen 10 und 11 schneiden sich an einer abgerundeten Kante 12, die gegenüber dem Zapfen 9 liegt. Diese beiden Zylinderflächen 10 und 11 bilden die einen Nocken zur Führung des Nockenkörpers 8. Diese Nocken bzw. Zylinderflächen 10 und 11 wirken mit den bogenförmigen Führungen 7, 7' zusammen.

Von den Seitenflächen des Nockenkörpers 8 springen Nocken 13 und 14 vor, die mit den bogenförmigen Führungen 6, 6' zusammenwirken. Diese Nocken 13 und 14 besitzen im gezeichneten Ausführungsbeispiel einen etwa ellipsenförmigen Querschnitt, wobei der eine Teil 15 mit dem größeren Krümmungsradius die dritte Seite des Bogendreieckes bildet und die beiden Teile 16 mit dem kleineren Krümmungsradius mit den bogenförmigen Führungen 6, 6' zusammenwirken.

Wie aus den Fig. 3 und 3a ersichtlich ist, liegt in der Streckstellung des Gelenkes die Zylinderfläche 10 an den Führungen 7 an und die Zylinderfläche 11 ist frei. Die Streckstellung wird überdies durch das Anliegen des Zapfens 9 an den Halbschalen 3 und 4 bestimmt. Um eine exakte Positionierung zu gewährleisten, liegt zweckmäßig der durch das Anliegen des Zapfens 9 an den Halbschalen 3 und 4 gebildete mechanische Anschlag etwa 1° vor dem durch die Zylinderfläche 10 und die Führung 7 gebildeten mechanischen Anschlag.

In der größten Schwenkstellung nach den Fig. 4 und Fig. 4a liegt der Zapfen 9 am gegenüberliegenden Ausschnittende der Halbschalen 3 und 4 an. In dieser Stellung ist die Zylinderfläche 10 frei und die Zylinderfläche 11 wirkt mit der Führung 7 zusammen. Auch hier ist es zweckmäßig, wenn der durch den Zapfen 9 und die Halbschalen 3 und 4 gebildete mechanische Anschlag etwa 1° vor dem durch die Zylinderfläche 11 und die Führung 7 gebildeten mechanischen Anschlag liegt.

Im Rahmen der Erfindung sind zahlreiche Abänderungen möglich. So kann ein erfindungsgemäßes Gelenk zufolge der freien Gestaltung der Steuereinheiten auch im Bereich von anderen Körpergelenken und Prothesen verwendet werden. Wird in anderen Gebieten eine kombinierte Dreh- und Gleitbewegung gefordert, kann auch dort ein erfindungsgemäßes Gelenk eingesetzt werden.

## Patentansprüche

1. Gelenk zur begrenzt schwenkbaren Lagerung zweier Schwenkteile, wobei mittels bogenförmiger Führungen (6,6'7,7') eine kombinierte Dreh- und Gleitbewegung erzeugt wird und wobei ein mit dem einen Schwenkteil verbundener Nockenkörper (8) Nocken aufweist, die mit den bogenförmigen Führungen zusammenwirken, die in Halbschalen (3,4) angeordnet sind, welche mit dem zweiten Schwenkteil verbunden sind, wobei der Nockenkörper (8) drei Nocken aufweist, wobei die zweite und dritte Nocke (13, 14) durch senkrecht zur Schwenkebene vorspringende Ansätze am Nockenkörper (8) gebildet werden, und wobei die Führungen (6, 6'; 7, 7') in den Halbschalen (3, 4) in zwei Stufen angeordnet sind, **dadurch gekennzeichnet daß** die erste Nocke durch an einer abgerundeten Kante (12) sich schneidende Zylinderflächen (10, 11) des Nockenkörpers (8) gebildet ist.

2. Gelenk nach Anspruch 1, **dadurch gekennzeichnet, daß** der Nockenkörper (8) im Querschnitt die Form eines Bogendreieckes mit abgerundeten Ecken besitzt.

3. Gelenk nach Anspruch 2, **dadurch gekennzeichnet, daß** die vorspringenden zweite und dritte Nocken (13, 14) an der der Schnittkante (12) der beiden Zylinderflächen (10, 11) gegenüberliegenden Seite des Bogendreieckes ausgebildet sind.

4. Gelenk nach einem der Ansprüche 1 bis 3, **dadurch gekennzeichnet, daß** der Anschluß eines Schwenkteiles gegenüberliegend der Schnittkante (12) der beiden Zylinderflächen (10, 11) angeordnet ist.

5. Gelenk nach einem der vorstehenden Ansprüche, **dadurch gekennzeichnet, daß** die beiden Halbschalen (3, 4) von einem Halteteil (1) teilweise umfaßt werden, an dem ein Anschluß für den anderen Schwenkteil angeordnet ist

6. Gelenk nach Anspruch 5, **dadurch gekennzeichnet, daß** die Halbschalen (3, 4) in den Halteteil (1) einschiebbar ausgebildet sind.

## Claims

1. A joint for holding, with limited hingeing movement, two hingeing parts, wherein by means of arc-shaped guides (6, 6'; 7, 7') a combined rotary and sliding movement is generated and wherein a cam body(8) connected to one hingeing part comprises cams which interact with the arc-shaped guides which are arranged in shells (3, 4), which are connected to the second hingeing part, wherein the cam body (8) comprises three cams, wherein the second and third cam (13, 14) are formed by projections on the cam body (8) which projections project perpendicular to the hingeing plane, and wherein the guides (6, 6'; 7, 7') are arranged in the shells (3, 4) in two steps, **characterised in that** the first cam is formed by cylinder surfaces (10, 11) of the cam body (8) which cylinder surfaces (10, 11) meet at a rounded edge (12).

2. The joint according to claim 1, **characterised in that** the cross-section of the cam body (8) has the shape of an arc-shaped triangle with rounded corners.

3. The joint according to claim 2, **characterised in that** the projecting second and third cams (13, 14) are arranged on the side of the arc-shaped triangle, which side is located opposite the edge of cut (12) of the two cylinder surfaces (10, 11).

4. The joint according to any one of claims I to 3, **characterised in that** the connection of a hingeing part is arranged opposite the edge of cut (12) of the two cylinder surfaces (10, 11).

5. The joint according to any one of the preceding claims, **characterised in that** the two shells (3, 4) are partly encompassed by a holding part (1) which comprises a connection for the other hingeing part.

6. The joint according to claim 5, **characterised in that** the shells (3, 4)

## Revendications

1. Articulation pour le logement pivotant limité de deux parties de pivotement, au moyen de guides en arceau (6, 6', 7, 7'), un mouvement combiné de rotation et de friction étant généré et un corps de came (8) relié à une pièce de pivotement présentant des cames qui coopèrent avec les guides en arceau, qui sont disposés dans des demi-coques (3, 4), lesquelles sont reliées à la seconde pièce de pivotement, le corps de came (8) présentant trois cames, les seconde et troisième cames (13, 14) étant formées sur le corps de came (8) par des butées faisant saillie perpendiculairement au plan de pivotement, et les guides (6, 6'; 7, 7') étant disposées sur deux étages dans les demi-coques (3, 4), **caractérisée en ce que** la première came est réalisée par des surfaces cylindriques (10, 11) du corps de came (8) se coupant au niveau d'un bord arrondi (12).

2. Articulation selon la revendication 1, **caractérisée en ce que** le corps de came (8) possède en section transversale la forme d'un triangle arqué avec des angles arrondis.

3. Articulation selon la revendication 2, **caractérisée en ce que** les seconde et troisième cames saillantes (13, 14) sont ménagées sur le côté du triangle arqué, opposé à l'arête de coupe (12) des deux surfaces cylindriques (10, 11).

4. Articulation selon l'une des revendications 1 à 3, **caractérisée en ce que** le raccordement d'une pièce de pivotement est disposé à l'opposé de l'arête de coupe (12) des deux surfaces cylindriques (10, 11).

5. Articulation selon l'une des articulations précédentes, **caractérisée en ce que** les deux demi-coques (3, 4) sont entourées partiellement par une pièce de retenue (1) sur laquelle est disposé un raccordement pour l'autre pièce de pivotement.

6. Articulation selon la revendication 5, **caractérisée en ce que** les demi-coques (3, 4) sont réalisées insérables dans la pièce de retenue (1).
